# EUROPEAN PATENT APPLICATION

(11) **EP 4 140 479 A1**
(43) Date of publication of application: **01.03.2023**
(21) Application number: 21792108.9
(22) Date of filing: 22.04.2021
(51) Int. Cl.: A61K 31/197, A61K 31/295, A61K 31/4985, A61K 33/06, A61K 33/24, A61K 33/26, A61K 33/30, A61K 45/00, A61P 31/14, A61P 43/00

(54) **THERAPEUTIC AND/OR PREVENTIVE AGENT FOR CORONAVIRUS DISEASE 2019 (COVID-19)**

(30) Priority: 22.04.2020 JP 2020076370; 16.10.2020 JP 2020174820
(71) Applicant: Neopharma Japan Co., Ltd., Shizuoka 437-0061 (JP); NAGASAKI UNIVERSITY, Nagasaki 852-8521 (JP)
(72) Inventor: KITA Kiyoshi, Nagasaki-shi, Nagasaki 852-8521 (JP); MORITA Kouichi, Nagasaki-shi, Nagasaki 852-8521 (JP); YASUDA Jiro, Nagasaki-shi, Nagasaki 852-8521 (JP); SAKURAI Yasuteru, Nagasaki-shi, Nagasaki 852-8521 (JP); KAWATA Satofumi, Tokyo 102-0071 (JP); TANAKA Tohru, Tokyo 102-0071 (JP); TOMIOKA Motoyasu, Tokyo 102-0071 (JP); FUJINE Kiyotaka, Tokyo 102-0071 (JP)
(74) Representative: Nederlandsch Octrooibureau
(86) International application number: PCT/JP2021/016372
(87) International publication number: WO 2021/215517

(57) **Abstract**

The present invention provides a therapeutic and/or preventive agent for coronavirus disease 2019 (COVID-19) comprising 5-aminolevulinic acid (ALA) or its derivative or a salt thereof and provides a method for treating and/or preventing coronavirus disease 2019 (COVID-19) using the therapeutic and/or preventive agent.

## Description

### Technical Field

The present invention relates to a therapeutic and/or preventive agent for coronavirus disease 2019 (COVID-19), and more particularly relates to a therapeutic and/or preventive agent for coronavirus disease 2019 (COVID-19) comprising 5-aminolevulinic acid (5-ALA) or its derivative or a salt thereof and relates to treatment and/or prevention of coronavirus disease 2019 (COVID-19) using the therapeutic and/or preventive agent.

### Background Art

Casesof coronavirus disease 2019 (COVID-19) were reported in December 2019. This disease is a novel pneumonia caused by severe acute respiratory syndrome coronavirus 2 (SARS-CoV-2). Severe acute respiratory syndrome coronavirus 2 (SARS-CoV-2) is a newly identified virus having similarities to SARS-CoV that causes the severe acute respiratory syndrome (SARS). Coronavirus disease 2019 (COVID-19) is known to cause, in addition to the pneumonia symptoms, a wide range of symptoms such as fever, cough, difficulty of breathing, dyspnea, chill, shivering with chills, muscular pain, headache, sore throat, dysgeusia, and dysosmia. The number of patients with coronavirus disease 2019 (COVID-19) is increasing rapidly around the world, and more than 150,000 deaths have been reported.

There is no specific therapy against severe acute respiratory syndrome coronavirus 2 (SARS-CoV-2) currently, and existing drugs against other viruses have been tested for their effects on severe acute respiratory syndrome coronavirus 2 (SARS-CoV-2). However, existing antiviral agents that show effects on severe acute respiratory syndrome coronavirus 2 (SARS-CoV-2) are limited (Non-Patent Literature 1). Therefore, development of an additional effective therapeutic agent against severe acute respiratory syndrome coronavirus 2 (SARS-CoV-2) has been awaited.

Furthermore, several variants of severe acute respiratory syndrome coronavirus 2 (SARS-CoV-2) have been recently detected, and it was pointed out that it may be more infectious and virulent than the conventional strain and/or a possibility of reducing the effects of immunity and vaccines to a lower level than the conventional strain. Thus, these variants have become new clinical problems (Non-Patent Literatures 3 and 4), and therefore development of additional therapeutic agents effective even against these variants has been longing for.

5-ALA is a common precursor of heme compounds produced in the mitochondria in cells, and it is known that, for example, 5-ALA, which is finally converted from protoporphyrin (PPIX) to heme, exhibits an anti-inflammatory effect on specific inflammatory diseases. Furthermore, it is known that PPIX accumulates in some cancer cells due to inhibition of conversion from PPIX to heme, and studies have been conducted to visualize cancer cells using the accumulated PPIX (Non-Patent Literature 2). However, it has not been known that 5-ALA and its derivative can inhibit infection, proliferation, and/or viral protein expression of severe acute respiratory syndrome coronavirus 2 (SARS-CoV-2) to treat or prevent coronavirus disease 2019 (COVID-19), and that combination with PPIX release inhibitor enhances the therapeutic or preventive effect.

### Citation List

### Non-Patent Literature

Non-Patent Literature 1: Cell Research, 2020, Vol.30, pp.269-271
Non-Patent Literature 2: Scientific Reports, 2019, Vol.9, pp.no.8666, doi: https://doi.org/10.1038/s41598-019-44981-y
Non-Patent Literature 3: Review in Medical Virology. 2021 Mar 16. doi: 10.1002/rmv.2231
Non-Patent Literature 4: the Ministry of Health, Labour and Welfare, "Response to Coronavirus Disease 2019 (Variants)" (URL:https://www.mhlw.go.jp/content/10900000/000766545.pdf, last accessed on April 20, 2021)

### Summary of Invention

### Technical Problem

An object of the present invention is to provide a therapeutic and/or preventive agent for coronavirus disease 2019 (COVID-19). More specifically, an object of the present invention is to provide a therapeutic and/or preventive agent for coronavirus disease 2019 (COVID-19) comprising 5-ALA or its derivative or a salt thereof. In another aspect, an object of the present invention is to provide a method for treating and/or preventing coronavirus disease 2019 (COVID-19) using 5-ALA or its derivative or a salt thereof. In another aspect, an object of the present invention is to provide a method for treating and/or preventing coronavirus disease 2019 (COVID-19) and to provide a medicine for use in the treatment and/or the prevention by using 5-ALA or its derivative or a salt thereof in combination with a PPIX release inhibitor. In another aspect, the present invention provides a method for inhibiting infection, proliferation, and/or expression of a viral nucleic acid or a viral protein of severe acute respiratory syndrome coronavirus 2 (SARS-CoV-2) and provides a medicine for use in the method by using 5-ALA or its derivative or a salt thereof preferably in combination with a PPIX release inhibitor.

### Solution to Problem

As a result of intensive studies to solve the above problems, the present inventors have found that, quite unexpectedly, 5-ALA inhibits infection, proliferation, and/or viral protein expression of severe acute respiratory syndrome coronavirus 2 (SARS-CoV-2), which is a causative virus of coronavirus disease 2019 (COVID-19), to treat or prevent COVID-19, and that 5-ALA has a synergistically excellent effect by concomitant use of a PPIX release inhibitor, and the present invention has been completed.

5-ALA is a common precursor of heme compounds produced in the mitochondria in cells. It has been known that, for example, 5-ALA exhibits an anti-inflammatory effect on specific inflammatory diseases, but it has not been known that 5-ALA and its derivative can inhibit infection, proliferation, and/or viral protein expression of severe acute respiratory syndrome coronavirus 2 (SARS-CoV-2) to treat or prevent coronavirus disease 2019 (COVID-19). The present invention newly provides a medicine comprising 5-ALA and its derivative for treatment or prevention of coronavirus disease 2019 (COVID-19), and provides use of the medicine.

Furthermore, the present inventors have also found that an extremely excellent therapeutic or preventive effect on coronavirus disease 2019 (COVID-19) can be obtained by using 5-ALA and a metal-containing compound such as sodium ferrous citrate (SFC) in combination.

That is, the present invention provides the following.

### [Item 1]

A therapeutic and/or preventive agent for coronavirus disease 2019 (COVID-19), the therapeutic and/or preventive agent comprising a compound or a salt of the compound, the compound represented by Formula (I) described below: 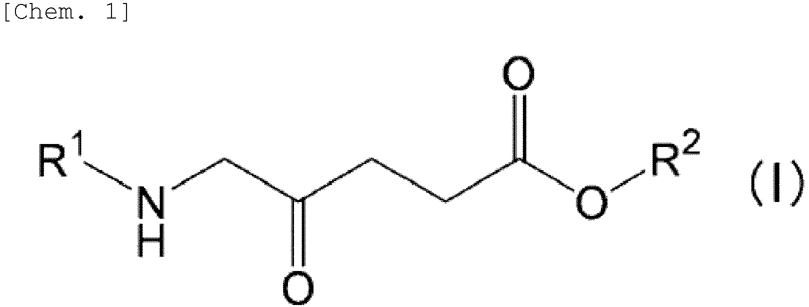 wherein R1 represents a hydrogen atom or an acyl group, and R2 represents a hydrogen atom, a linear or branched alkyl group, a cycloalkyl group, an aryl group, or an aralkyl group.

### [Item 2]

The therapeutic and/or preventive agent for coronavirus disease 2019 (COVID-19) according to Item 1, wherein R1 and R2 are respectively a hydrogen atom.

### [Item 3]

The therapeutic and/or preventive agent for coronavirus disease 2019 (COVID-19) according to Item 1 or 2, further comprising one or more metal-containing compounds.

### [Item 4]

The therapeutic and/or preventive agent for coronavirus disease 2019 (COVID-19) according to Item 3, wherein the one or more metal-containing compounds are a compound containing iron, magnesium, zinc, nickel, vanadium, copper, chromium, molybdenum, or cobalt.

### [Item 5]

The therapeutic and/or preventive agent for coronavirus disease 2019 (COVID-19) according to Item 3, wherein the one or more metal-containing compounds are a compound containing iron, magnesium, or zinc.

### [Item 6]

The therapeutic and/or preventive agent for coronavirus disease 2019 (COVID-19) according to Item 3, wherein the one or more metal-containing compounds are a compound containing iron.

### [Item 7]

The therapeutic and/or preventive agent for coronavirus disease 2019 (COVID-19) according to any one of Items 1 to 6, the therapeutic and/or preventive agent to be used in combination with a PPIX release inhibitor.

### [Item 8]

The therapeutic and/or preventive agent for coronavirus disease 2019 (COVID-19) according to Item 7, wherein the PPIX release inhibitor is a transporter inhibitor or an exocytosis inhibitor.

### [Item 9]

The therapeutic and/or preventive agent for coronavirus disease 2019 (COVID-19) according to Item 7 or 8, wherein the PPIX release inhibitor is an ABCG2 inhibitor or a dynamin inhibitor.

### [Item 10]

The therapeutic and/or preventive agent for coronavirus disease 2019 (COVID-19) according to any one of Items 7 to 9, wherein the PPIX release inhibitor is an ABCG2 inhibitor.

### [Item 11]

A method for treating and/or preventing coronavirus disease 2019 (COVID-19), the method including administering a compound or a salt of the compound with a pharmaceutically acceptable excipient, the compound represented by Formula (I) described below: 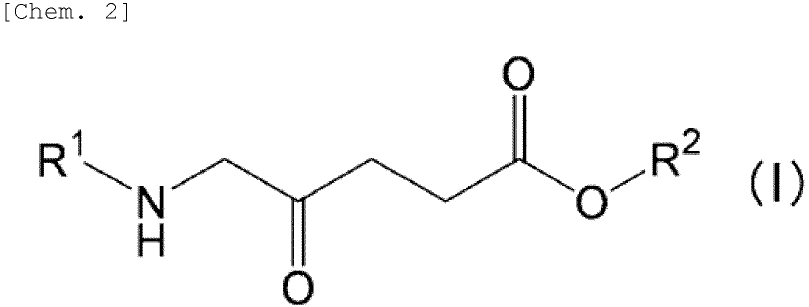 wherein R¹ represents a hydrogen atom or an acyl group, and R² represents a hydrogen atom, a linear or branched alkyl group, a cycloalkyl group, an aryl group, or an aralkyl group.

### [Item 12]

Use of a compound or a salt of the compound for treatment and/or prevention of coronavirus disease 2019 (COVID-19), the compound represented by Formula (I) described below: 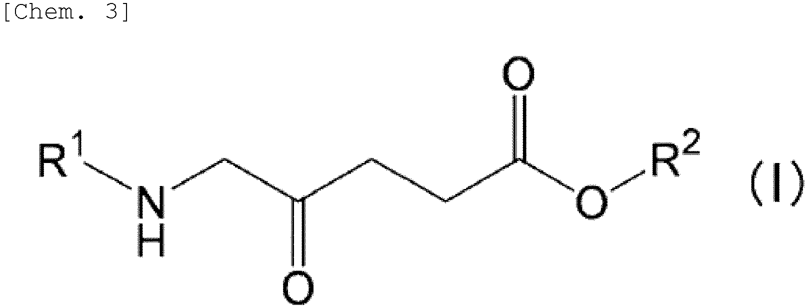 wherein R¹ represents a hydrogen atom or an acyl group, and R² represents a hydrogen atom, a linear or branched alkyl group, a cycloalkyl group, an aryl group, or an aralkyl group.

### Advantageous Effects of Invention

The therapeutic and/or preventive agent for coronavirus disease 2019 (COVID-19) of the present invention strongly inhibits, particularly cell infection or proliferation of the virus, expression or assembly of viral capsid proteins, and/or release of virus particles, and thus inhibits an increase of infected cells and exhibits an excellent therapeutic and/or preventive effect on coronavirus disease 2019 (COVID-19). In another aspect, the method for treating and/or preventing coronavirus disease 2019 (COVID-19) of the present invention in which 5-aminolevulinic acid (5-ALA) or its derivative or a salt thereof are used in combination with a PPIX release inhibitor, and concerned treatment and/or medicine to be provided to have effect in the treatment and/or prevention of coronavirus disease 2019 (COVID-19).

### Brief Description of Drawings

Fig. 1 is a graph showing an effect of 5-ALA and FTC on SARS-CoV-2 infection in VeroE6 cells.
Fig. 2 is a graph showing an effect of 5-ALA and FTC on SARS-CoV-2 infection in VeroE6 cells.
Fig. 3 is a graph showing an effect of 5-ALA, or 5-ALA and SFC on SARS-CoV-2 infection in Caco-2 cells.
Fig. 4 is a graph showing an effect of 5-ALA, or 5-ALA and SFC on SARS-CoV-2 infection in Caco-2 cells.
Fig. 5 is a graph showing a concentration-dependent infection inhibitory effect and a cytotoxic effect of application of 5-ALA, 5-ALA and SFC, or SFC alone on SARS-CoV-2 infection in VeroE6 cells.
Fig. 6 is a graph showing a concentration-dependent infection inhibitory effect and a cytotoxic effect of application of 5-ALA, 5-ALA and SFC, or SFC alone on SARS-CoV-2 infection in Caco-2 cells.

### Description of Embodiments

### (Definitions)

In the present description, when a plurality of numerical ranges are indicated, a range consisting of a combination of any of the lower limit value and upper limit value of the plurality of ranges is also meant as well.

### (Severe Acute Respiratory Syndrome Coronavirus 2 (SARS-CoV-2))

The severe acute respiratory syndrome coronavirus 2 (SARS-CoV-2) as a subject of the present invention includes not only the conventional strain but also variants (see Non-Patent Literature 3 and 4, and the like). Examples of the "variant having a mutation of N501Y" include a variant observed in the United Kingdom (VOC-202012/01), a variant observed in South Africa (501Y.V2), a variant observed in Brazil (501Y.V3), and a variant observed in the Philippines, and examples of the "variant having a mutation of E484K" include a variant observed in South Africa (501Y.V2), a variant observed in Brazil (501Y.V3), and a variant observed in the Philippines, but the variants are not limited thereto.

### (Active Substances of Therapeutic and/or Preventive Agent for Coronavirus Disease 2019 (COVID-19) and Protein Expression Inhibitor for Severe Acute Respiratory Syndrome Coronavirus 2 (SARS-CoV-2) of the Present Invention)

A compound to be used as an active substance of the therapeutic and/or preventive agent for coronavirus disease 2019 (COVID-19) and protein expression inhibitor for the severe acute respiratory syndrome coronavirus 2 (SARS-CoV-2) of the present invention can be exemplified by a compound represented by Formula (I) or its salt (hereinafter, these compounds may be collectively referred to as "ALAs"). 5-ALA, which is also referred to as δ-aminolevulinic acid, is represented by Formula (I) in which both R¹ and R² are both hydrogen atoms, and is a kind of amino acid. Examples of the 5-ALA derivative include compounds, other than 5-ALA, are represented by Formula (I) in which R¹ is a hydrogen atom or an acyl group and R² is a hydrogen atom, a linear or branched alkyl group, a cycloalkyl group, an aryl group, or an aralkyl group.

Examples of the acyl group in Formula (I) include linear or branched alkanoyl groups having 1 to 8 carbon atoms, such as formyl, acetyl, propionyl, butyryl, isobutyryl, valeryl, isovaleryl, pivaloyl, hexanoyl, octanoyl, and benzylcarbonyl groups, and aroyl groups having 7 to 14 carbon atoms, such as benzoyl, 1-naphthoyl, and 2-naphthoyl groups.

Examples of the alkyl group in Formula (I) include linear or branched alkyl groups having 1 to 8 carbon atoms, such as methyl, ethyl, propyl, isopropyl, butyl, isobutyl, sec-butyl, tert-butyl, pentyl, isopentyl, neopentyl, hexyl, heptyl, and octyl groups.

Examples of the cycloalkyl group in Formula (I) include cycloalkyl groups having 3 to 8 carbon atoms in which a saturated or partially unsaturated bond may be present, such as cyclopropyl, cyclobutyl, cyclopentyl, cyclohexyl, cycloheptyl, cyclooctyl, cyclododecyl, and 1-cyclohexenyl groups.

Examples of the aryl group in Formula (I) include aryl groups having 6 to 14 carbon atoms, such as phenyl, naphthyl, anthryl, and phenanthryl groups.

The aralkyl group in Formula (I) can have an aryl moiety exemplified in the same manner as the above-described aryl group, and can have an alkyl moiety exemplified in the same manner as the above-described alkyl group. Specific examples of the aralkyl group include aralkyl groups having 7 to 15 carbon atoms, such as benzyl, phenethyl, phenylpropyl, phenylbutyl, benzhydryl, trityl, naphthylmethyl, and naphthylethyl groups.

The 5-ALA derivative is preferably a compound in which R¹ is a group such as a formyl, acetyl, propionyl, or butyryl group, or a compound in which R² is a group such as a methyl, ethyl, propyl, butyl, or pentyl group, and preferred examples of the 5-ALA derivative include compounds including a combination of formyl and methyl, acetyl and methyl, propionyl and methyl, butyryl and methyl, formyl and ethyl, acetyl and ethyl, propionyl and ethyl, or butyryl and ethyl as a combination of R¹ and R² described above.

The 5-ALAs are to act as an active substance in a state of 5-ALA of Formula (I) or its derivative in vivo, and are to be administered, according to the dosage form, in a form of a salt or an ester for improvement in solubility or in a form of a prodrug (precursor) to be degraded by an enzyme in vivo. Examples of the salts of 5-ALA and its derivative include pharmacologically acceptable acid addition salts, metal salts, ammonium salts, and organic amine addition salts. Examples of the acid addition salts include inorganic acid salts such as hydrochlorides, hydrobromides, hydroiodides, phosphates, nitrates, and sulfates, and organic acid addition salts such as formates, acetates, propionates, toluenesulfonates, succinates, oxalates, lactates, tartrates, glycolates, methanesulfonates, butyrates, valerates, citrates, fumarates, maleates, and malates. Examples of the metal salts include alkali metal salts such as lithium salts, sodium salts, and potassium salts, alkaline earth metal salts such as magnesium salts and calcium salts, and salts of a metal such as aluminum or zinc. Examples of the ammonium salts include alkylammonium salts such as ammonium salts and tetramethylammonium salts. Examples of the organic amine salt include salts such as triethylamine salts, piperidine salts, morpholine salts, and toluidine salts. These salts can also be used in a form of a solution at the time of use.

Among the above ALAs, desirable ALAs are 5-ALA, esters such as 5-ALA methyl ester, 5-ALA ethyl ester, 5-ALA propyl ester, 5-ALA butyl ester, and 5-ALA pentyl ester, and hydrochlorides, phosphates, and sulfates thereof. ALA hydrochloride and 5-ALA phosphate can be particularly preferred examples.

The ALAs can be produced byany known method of chemical synthesis, production by a microorganism, and production by an enzyme. The ALAs may form a hydrate or a solvate. And, any of them can be used aloneor in an appropriate combination of two or more kinds thereof.

The therapeutic and/or preventive agent for coronavirus disease 2019 (COVID-19) and protein expression inhibitor for the severe acute respiratory syndrome coronavirus 2 (SARS-CoV-2) of the present invention preferably further comprises a metal-containing compound as long as an overload disorder does not occur. Examples of the metal moiety of the metal-containing compound include iron, magnesium, zinc, nickel, vanadium, cobalt, copper, chromium, and molybdenum. Iron, magnesium, and zinc are preferable, and iron can be a particularly preferred example.

An effect of inhibiting infection, proliferation, and/or viral protein expression of severe acute respiratory syndrome coronavirus 2 (SARS-CoV-2) and an excellent effect of treating and/or preventing coronavirus disease 2019 (COVID-19) can be obtained by using the ALAs in combination with the metal-containing compound even if the ALAs are used at a further low concentration.

The iron compound may be an organic salt or an inorganic salt. Examples of the inorganic salt include ferric chloride, iron sesquioxide, iron sulfate, and ferrous pyrophosphate, and examples of the organic salt include carboxylates, for example, citrates such as ferrous citrate, ferric sodium citrate, sodium ferrous citrate (SFC), and ammonium iron citrate, organic salts such as ferric pyrophosphate, heme iron, iron dextran, iron lactate, ferrous gluconate, iron sodium diethylenetriaminepentaacetate, iron ammonium diethylenetriaminepentaacetate, iron sodium ethylenediaminetetraacetate, iron ammonium ethylenediaminepentaacetate, iron sodium dicarboxymethylglutamate, iron ammonium dicarboxymethylglutamate, ferrous fumarate, iron acetate, iron oxalate, ferrous succinate, and iron sodium succinate citrate, iron triethylenetetraamine, lactoferrin iron, transferrin iron, sodium iron chlorophyllin, ferritin iron, saccharated iron oxide, and ferrous glycine sulfate, which are hydroxycarboxylates.

Examples of the magnesium compound include magnesium citrate, magnesium benzoate, magnesium acetate, magnesium oxide, magnesium chloride, magnesium hydroxide, magnesium carbonate, magnesium sulfate, magnesium silicate, magnesium nitrate, magnesium diammonium diethylenetriaminepentaacetate, magnesium disodium ethylenediaminetetraacetate, and magnesium protoporphyrin.

Examples of the zinc compound include zinc chloride, zinc oxide, zinc nitrate, zinc carbonate, zinc sulfate, zinc diammonium diethylenetriaminepentaacetate, zinc disodium ethylenediaminetetraacetate, zinc protoporphyrin, and zinc-containing yeast.

As the metal-containing compound, a compound including one or more kinds of the above-described metal-containing compounds can be used, and the dose of the metal-containing compound is to be 0 to 100 times, desirably 0.01 times to 10 times, and more desirably 0.1 times to 8 times the dose of 5-ALA in terms of molar ratio.

The therapeutic and/or preventive agent for coronavirus disease 2019 (COVID-19) of the present invention can be used in combination with another antiviral agent or another therapeutic agent for a disease as long as an overload disorder does not occur. The dose of such another agent and the ratio of the dose between another agent and 5-ALA can be appropriately adjusted by those skilled in the art.

The ALAs of the present invention are preferably used in combination with a PPIX release inhibitor. As the PPIX release inhibitor, a transporter inhibitor or an exocytosis inhibitor can be used, and as these inhibitors, for example, a multi-drug resistant transporter inhibitor such as an ABCG2 inhibitor, a dynamin inhibitor, a chelating agent, and an activator of vitamin D can be used.

The specific PPIX release inhibitor used in the present invention is not particularly limited. Examples of the PPIX release inhibitor include an ABCG2 inhibitor, a chelating agent, and an activator of vitamin D. The fact that a chelating agent can be used for increasing the accumulation of PpIX in a cell is described in, for example, WO 2014/2023833 and Photochem Photobiol. 2010, Vol. 86, no. 2, pp. 471-475. Furthermore, the fact that an activator of vitamin D can be used for increasing the accumulation of PpIX in a cell is described in, for example, Cancer Res, 2011, Vol, 71, no. 18, pp. 6040-6050.

ABCG2 inhibitors that may be optionally used in the present invention are, for example, compounds described in Tables 3 and 4 in Int J Biochem Mol Biol, 2012, Vol. 3, no. 1, pp. 1-27, compounds described in Table 2 in Chin J Cancer, 2012, Vol. 31, no. 2, p75-99, topoisomerase II inhibitor (Mitoxantrone, Bisantrene, Etoposide, Becatecarin, NB-506, J-107088, and the like), Anthracyclines (Daunorubicin, Doxobucincin, Epirubicin, Pirarubicin, and the like), Camptothecin analogs (topoisomerase I inhibitor) (Topotecan, SN-38, CPT-11, 9-aminocamptothecin, NX211, DX-8951f, Homocamptothecins, BN80915 (diflomotecan), Gimatecan, Belotecan, and the like), tyrosine kinase inhibitors (Dasatinib, Vandetanib, Nilotinib, Sorafenib, Tandutinib, CI1033 (Pan-HER TKI), CP-724,714 (HER2 TKI), Symadex (fms-like tyrosine kinase 3 inhibitor), and the like), Antimetabolites (MTX, MTX diglutamate, MTX triglutamate (antifolate), GW1843, Tomudex (antifolates), Trimetrexatte, piritrexim, metoprine, pyrimethamine (lipophilic antifolates), 5-fluorouracil (pyrimidine analog), CdAMP (nucleotide), cladribine (nucleoside), and the like), cyclin-dependent kinase inhibitor (Flavopiridol and the like), CDK and aurora kinases inhibitor (JNJ-7706621 and the like), non-steroidal anti-androgen (Bicalutamide and the like), PEITC (Phenethyl isothiocyanate and the like), indazole-based tubulin inhibitors (TH-337 and the like), Sufate and glucuronide conjugates of xenobiotics (Estrone 3-sulfate (E1S), 17beta-estradiol sulfate, DHEAS, 4[35S]-methylumbelliferone sulfate, E3040 sulfate, Troglitazone sulfate, 3-O-sulfate conjugate of 17alpha-ethinylestradiol, SN-38-glucuronide, [3H]17beta-estradiol-17beta-D-glucuronide, [14C]4-methylumbelliferone glucuronide, BP-3-sulfate, BP-3-glucuronide, Phenolic MPA glucuronide, and the like), natural compounds and toxins (folic acid, urate, genistein, riboflavin (vitamin B2), plumbagin (vitamin K3), glutathione (GSH), sphingosine 1-phosphate, PhIP (carcinogen), and the like), and other compounds ([(125)I]lodoarylazidoprazosin (IAAP), [(3)H]azidopine; Sulfasalazine (anti-inflammatory); Erythromycin (macrolide antibiotic); Ciprofloxacin, ofloxacin, norfloxacin, enrofloxacin, grepafloxacin, ulifloxacin (fluoroquinolone antibiotics); Nitrofurantoin (urinary tract antibiotic); Moxidectin (parasiticide); Albendazole suloxide and oxfendazole (anthelmintics); Ganciclovir (antiviral drug); Zidovudine, Lamivudine (NRTI); Leflunomide, A771726 (antirheumatic drugs); Diclofenac (analgesic and anti-inflammatory drug); Cimetidine (histamine H2-receptor antagonist); ME3277 (hydrophilic glycoprotein IIb/IIIa antagonist); Pitavastatin, Rosuvastatin (HMG-CoA reductase inhibitor); Dipyridamole (thromboxane synthase inhibitor); Glyburide (hypoglycemic agent); Nicardipine, nifedipine, nitrendipine (Ca2+ channel blocker); Olmesartan medoxomil (angiotensin II AT1-R antagonist); Befloxatone (selective monoamine oxidase inhibitor); Prazosin (alpha-1-adrenergic receptor antagonist); Riluzole (Na+ channels blocker); Amyloid-beta Zoledronic acid (osteotropic compound); Hesperetin conjugates, Kaempferol (flavonoid, see also WO 2004/069233); FTC (Fumitremorgin C) and its derivative (see Mol. Cancer Ther., 2002, 1: 417-425); estrogen and antiestrogen (see Mol. Cancer Ther., 2003, 2: 105-112); novobiocin (see Int. J. Cancer, 2004, 108: 146-151); a diphenylacrylonitrile derivative (see WO 2004/069243); acrylonitrile derivatives having a heterocyclic ring (see WO 2006/106778, WO 2009/072267).

As the PPIX release inhibitor, a compound including one or more kinds of the above-described PPIX release inhibitors can be used, and the dose of the PPIX release inhibitor can be appropriately selected according to the desired therapeutic effect. For example, the PPIX release inhibitor can be used in a dose of 0 to 10000 times, preferably 0.0 times to 1000 times, more preferably 1 time to 300 times, and still more preferably 10 to 100 times the dose of 5-ALA in terms of molar ratio.

### (Method of Administration of Therapeutic and/or Preventive Agent of the Present Invention)

In the method for treating and/or preventing coronavirus disease 2019 (COVID-19) of the present invention or in the medicine to be used in these methods, the ALAs, the PPIX release inhibitor, the metal-containing compound, or another agent can be administered as a composition containing all of them, or as a composition containing each aloneor containing only a part of them. In one aspect, in the case of using compositions containing the ALAs and other agents respectively alone or containing only a part of the ALAs and other agents, these compositions may be administered simultaneously or separately. The compositions can be preferably administered in combination so that the administration of the ALAs and other agents can exhibit an additive effect or a synergistic effect. In the case of administering the ALAs and other agents individually or administering compositions each containing only a part of the ALAs and other agents separately, the administration interval thereof can be set to 5 minutes, 10 minutes, 15 minutes, 20 minutes, 30 minutes, 45 minutes, 1 hour, 2 hours, 3 hours, 4 hours, 5 hours, 6 hours, 7 hours, 8 hours, 9 hours, 10 hours, 11 hours, 12 hours, or the like, but is not limited thereto.

The route of administration of the therapeutic and/or preventive agent for coronavirus disease 2019 (COVID-19) of the present invention is not particularly limited, but administration can be used such as oral administration, inhalation administration, intravenous administration by injection, drip infusion, or the like, transdermal administration, or parenteral administration such as administration by a suppository or administration by forced enteral feeding using a nasogastric tube, a nasoenteric tube, a gastric fistula tube, or an enteric fistula tube.

### (Dosage Form of Therapeutic and/or Preventive Agent of the Present Invention)

The dosage form of the therapeutic and/or preventive agent for coronavirus disease 2019 (COVID-19) of the present invention can be appropriately determined according to the administration route described above, and examples of the dosage form include injection drugs, drops, tablets, capsules, fine granules, powders, liquids, water agents by dissolving in a syrup or the like, cataplasms, and suppositories.

In order to prepare the therapeutic and/or preventive agent for coronavirus disease 2019 (COVID-19) of the present invention, pharmacologically acceptable carriers, excipients, diluents, additives, disintegrants, binders, covering agents, lubricants, glidants, lubricants, flavoring agents, sweeteners, solubilizing agents, solvents, gelling agents, and nutrients and the like can be added as necessary, and those skilled in the art can select a specific substance according to the purpose.

### (Dose of Therapeutic and/or Preventive Agent of the Present Invention)

The dose, the administration frequency, and the administration period of the therapeutic and/or preventive agent for coronavirus disease 2019 (COVID-19) of the present invention depend on the specie of the animal, the age, the body weight, the symptom, and the like in which coronavirus disease 2019 (COVID-19) is to be treated or prevented. For example, in the case of administering the agent to a human, the dose of the ALAs can be 0.01 to 200 mg/kg body weight/day, preferably 0.5 to 100 mg/kg body weight/day, more preferably 1 to 50 mg/kg body weight/day, and still more preferably 5 to 30 mg/kg body weight/day in terms of the weight of 5-ALA. Particularly in the case of using the agent as a preventive agent, the agent is desirably taken in a low dose continuously. For example, in the case of administering the agent as a preventive supplement, the agent can be administered in a dose of, for example, 0.1 to 30 mg/kg body weight/day, preferably 0.3 to 10 mg/kg body weight/day, and more preferably 1.0 to 5 mg/kg body weight/day. The administration frequency can be, for example, once to multiple times of administration per day or continuous administration by drip infusion or the like. The period of administration as a therapeutic agent or a preventive agent can be determined according to known methods by an expert in the art, such as a doctor and the like.

### (Indication for Therapeutic and/or Preventive Agent of the Present Invention)

The therapeutic and/or preventive agent for coronavirus disease 2019 (COVID-19) of the present invention can be applied to treatment and prevention of various symptoms resulting from infection with severe acute respiratory syndrome coronavirus 2 (SARS-CoV-2) and/or onset of coronavirus disease 2019 (COVID-19). Examples of the indication include, but are not limited to, pneumonia, fever, cough, difficulty of breathing, dyspnea, chill, shivering with a chill, muscular pain, headache, sore throat, dysgeusia, and dysosmia.

### (Animal to which Therapeutic and/or Preventive Agent of the Present Invention is Applied)

The therapeutic and/or preventive agent for coronavirus disease 2019 (COVID-19) of the present invention can be administered to humans, monkeys, dogs, cats, minks, cows, pigs, related animals thereof, other mammals, birds, and the like.

Hereinafter, the present invention will be described in more detail with reference to Examples, but the present invention is not limited thereto.

### Example 1

### (Treatment Example)

1. Patient: 1 male, 53 years old
2. Treatment course

Since the night of Friday, February 28, 2020, the patient felt unusually strong malaise, a chill, a runny nose, a sore throat, and a chest pain, and had dry cough. When the patient woke up at night and measured the body temperature, and the body temperature was 37.5°C. The patient took orange juice to quench his thirst, but he felt no taste.

After a sleepless night due to the intolerable malaise, the patient began to take a supplement (capsule) containing 50 mg of 5-ALA phosphate and 28.7 mg of SFC on Saturday, February 29, and the following course was observed.
Saturday, February 29
2:00 As a result of intake of 4 capsules, the chill was improved, and the body temperature was 37.5°C.
4:00 The patient felt a chill again,and took 2 capsules, and the body temperature was 37.2°C.
5:00 The patient still had a cough and a sneeze, but the throat pain was reduced, and the body temperature was 36.8°C.
8:00 Since the body temperature rose to 37.9°C, the patient took 4 capsules again, and the body temperature went down to 37.2.
10:00 The body temperature was 37.5°C, the patient took 2 capsules, and the body temperature went down to 37.1°C.
12:00 The body temperature was 37.4°C, the patient took 2 capsules, and the body temperature went down to 37.0°C.
15:00 The body temperature was 37.2°C, the patient took 2 capsules, and the body temperature got normal to 36.8°C and the malaise started to improve.
19:00 The body temperature was 36.9°C, the patient was able to take dinner, the taste was normalized, and the patient took 2 capsules and slept.
Sunday, March 1
5:00 The body temperature was 36.8°C, the patient took 4 capsules, and the cold symptoms were ameliorated.
10:00 The body temperature was 36.9°C, the patient took 4 capsules, and had no symptoms.
12:00 The body temperature was 36.8°C, the patient took 4 capsules, and had no symptoms.
16:00 The body temperature was 36.8°C, the patient felt a mild muscular pain and took 4 capsules.
17:30 The muscular pain was ameliorated, the body temperature was 36.9°C, and the patient had no symptoms.

Thereafter, on April 17, the male patient was examined for an anti-novel coronavirus antibody using a severe acute respiratory syndrome coronavirus 2 (SARS-CoV-2) antibody test reagent kit (Kurabo Industries Ltd., product code RF-NC002), and as a result, the test was positive.

### 3. Results

From the above course, it has become clear that various symptoms of coronavirus disease 2019 (COVID-19) can be remarkably improved in a short period by intake of 5-ALA.

### Example 2

### (Cell Experiment)

### (Method)

### Materials

VeroE6, a cell line established from African green monkey kidney, was obtained from Dr. Takada of Hokkaido University. Caco-2 cells derived from human colon cancer were obtained from Dr. Iida at Osaka University.

The VeroE6, a cell line established from African green monkey kidney were cultured in Dulbecco's modified Eagle medium (DMEM) supplemented with 10% fetal bovine serum (FBS) and 1% penicillin/streptomycin.

Caco-2 cells were also cultured in a similar manner.

A 100 mM solution of 5-aminolevulinic acid (5-ALA) hydrochloride was prepared with pure water, a 25 mM solution of sodium ferrous citrate (SFC) was prepared with pure water and 1 N HCl, and 5 mM fumitremorgin C (FTC) was prepared with DMSO, and these solutions were diluted with DMEM and used for each experiment. For the immunostaining method, goat serum, rabbit anti-SARS-CoV N antibody (Novus Biologicals, LLC), Alexa Fluor 488 goat anti-rabbit (Thermo Fisher Scientific K.K.), and Hoechst 33342 (Thermo Fisher Scientific K.K.) were used.

### Virus

The SARS-CoV-2 used in this experiment was isolated from an infected person in Japan (A JPN/NGS/IA-1/2020, GISAID accession no. EPI-ISL-481251). The variants of SARS-CoV-2 used were 6 strains obtained from National Institute of Infectious Diseases: a UK strain QK002 (hCoV-19/Japan/QK002/2020, GISAID ID: EPI_ISL_768526), a UK strain QHN001 (hCoV-19/Japan/QHN001/2020, GISAID ID: EPI_ISL_804007), a UK strain QHN002 (hCoV-19/Japan/QHN002/2020, GISAID ID: EPI_ISL_804008), a Brazil strain TY7-501 (hCoV-19/Japan/TY7-501/2021, GISAID ID: EPI_ISL_833366), a Brazil strain TY7-503 (hCoV-19/Japan/TY7-503/2021, GISAID ID: EPI_ISL_877769), and a South Africa strain TY8-612 (hCoV-19/Japan/TY8-612/2021, GISAID ID: EPI_ISL_1123289). The virus was passaged using VeroE6 cells and cultured for 3 to 4 days, then the culture supernatant was collected and centrifuged at 2000 x g for 15 minutes, and then the supernatant was stored at -80°C, thawed for each experiment, and then used. All of the infection experiments using this virus were performed in the BSL3 laboratory of Nagasaki University.

### Test for Evaluation of Efficacy

### (a) Test with VeroE6 Cells

VeroE6 cells were seeded in 96-well plate at a density of 0.5 x 10^4 cells/well, and after the cells adhered to the bottom of the plate, each agent was added to the medium. The test agents used were 5-ALA (1000 uM), 5-ALA (1000 uM) + FTC (10 uM), 5-ALA (1000 uM) + SFC (250 uM), 5-ALA (1000 uM) + SFC (250 uM) + FTC (10 uM), and FTC (10 uM). After adding the agents, the cells were cultured at 37°C for 48 hours or 72 hours. The cells were then infected with SARS-CoV-2 in the BSL3 laboratory. After 48 hours from the start of infection, the infected cells were immersed in 4% paraformaldehyde (PFA) overnight to fix the cells and inactivate the virus. The cells were permeabilized with 0.2% Triton X-100, then blocked with 100 goat serum, treated with rabbit anti-SARS-CoV N antibody as a primary antibody, and treated with Alexa Fluor 488 goat anti-rabbit as a secondary antibody, and the cell nuclei were stained with Hoechst 33342. Thereafter, images of the infected cells were taken using a Cytation 5 cell imaging plate reader (BioTek Instruments). The images were analyzed using CellProfiler image analysis software (Broad Institute of MIT)to calculate the number of the infected cells and the total number of the cells, and graphing and statistical processing were performed using GraphPad Prism (MDF Co., Ltd.).

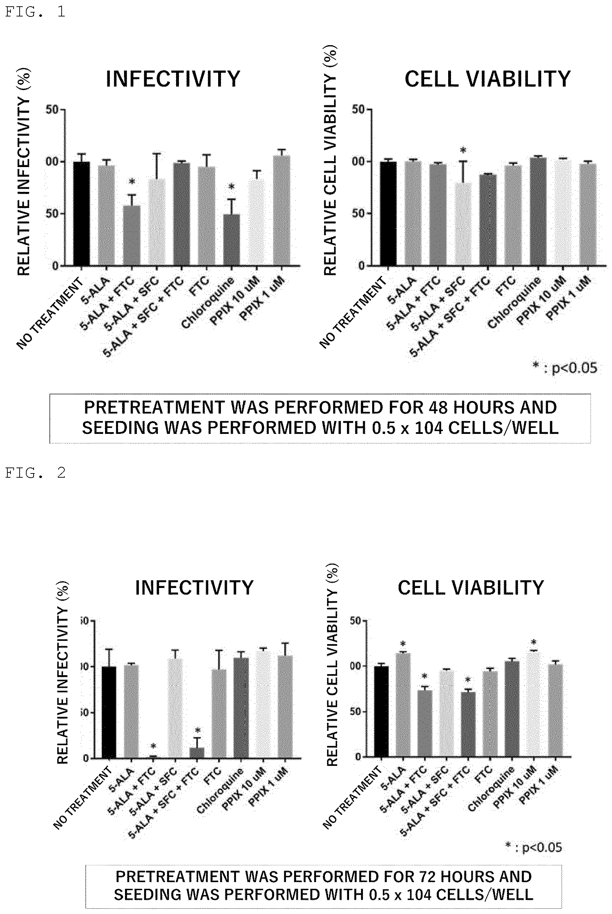

Among the conditions where pretreatment was performed for 48 hours with an agent, infection with SARS-CoV-2 was significantly inhibited under the condition of adding 5-ALA and FTC. No effect was observed under other conditions of adding an agent (Fig. 1). Among the conditions where pretreatment was performed for 72 hours with an agent, infection with SARS-CoV-2 was strongly inhibited under the condition of adding 5-ALA and FTC and under the condition of adding 5-ALA, SFC, and FTC (Fig. 2).

From the above results, it was found that the antiviral activity was enhanced by adding 5-ALA and FTC or by adding 5-ALA, SFC, and FTC.

Furthermore, in a case where the number of seeded cells was set to 1 × 10^4 cells/well, infection with SARS-CoV-2 was significantly inhibited by adding 5-ALA alone or by adding 5-ALA and SFC.

### (b) Test with Caco-2 Cells

Drug evaluation test was performed using Caco-2 cells in the same manner except that the time from infection with SARS-CoV-2 to inactivation was changed to 72 hours and that the amount of virus with which the cells were infected was 0.02 MOI in the Caco-2 cells, whereas the amount was 0.002 MOI in the VeroE6 cells.

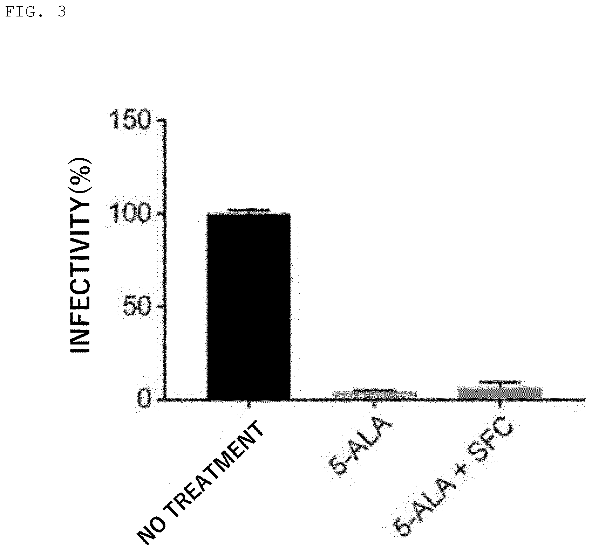

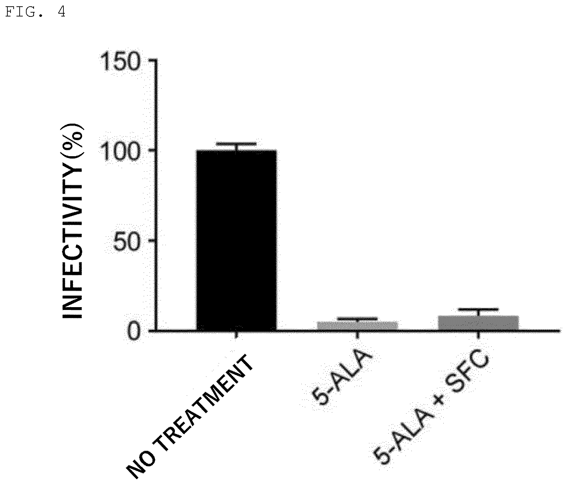

In a case where an agent was added and then the cells were cultured for 72 hours and then infected with SARS-CoV-2, SARS-CoV-2 infection and/or proliferation was strongly inhibited in cells treated with 5-ALA alone or in cells treated with 5ALA and SFC in combination (Fig. 3). In Caco-2 cells, even in a case where cells were cultured for 48 hours after addition of each agent followed by infection with SARS-CoV-2, a similar inhibitory effect on SARS-CoV-2 infection and/or proliferation was obtained (Fig. 4) .

Furthermore, even in a case where cells were infected with a variant in the same manner, an inhibitory effect on SARS-CoV-2 infection and/or proliferation is expected in cells treated with 5-ALA alone or in cells treated with 5ALA and SFC in combination.

These results confirm that 5-ALA exhibits an inhibitory effect on SARS-CoV-2 infection and/or proliferation in human cells.

Caco-2 cells are known to metabolize 5-ALA supplied from the outside of cells (Biochem. Biophys. Rep., 2017. Vol/11, pp. 105-111).

### (c) Dose-Dependent Inhibitory Effect on Infection and/or Proliferation

In order to confirm the dose dependence in the inhibitory effect on SARS-CoV-2 infection and/or proliferation, the agents at each concentration were tested.

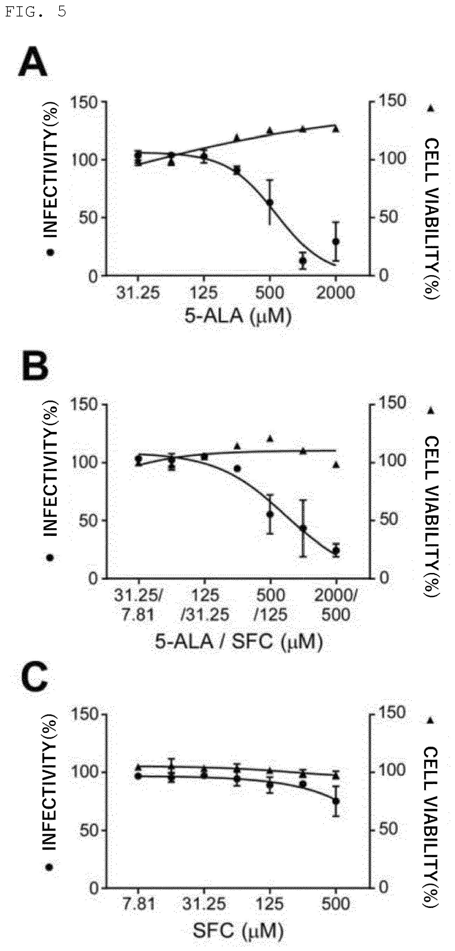

As a result, in VeroE6 cells, the IC₅₀ of 5-ALA alone was 570 uM, and the IC₅₀ of 5-ALA and SFC in combination was 695 uM. Meanwhile, application of SFC alone showed no inhibitory effect on SARS-CoV-2 infection and/or proliferation (Fig. 5).

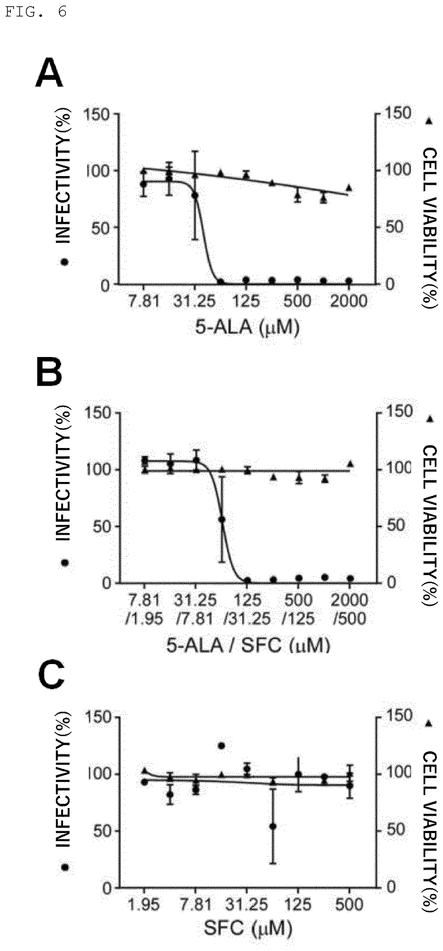

In Caco-2 cells, the IC₅₀ of 5-ALA alone was 39 uM, and the IC₅₀ of 5-ALA and SFC in combination was 63 uM. Meanwhile, application of SFC alone showed no inhibitory effect on SARS-CoV-2 infection and/or proliferation (Fig. 6) .

Note that in all concentration ranges tested (up to 2000 uM), 5-ALA did not show significant cytotoxicity.

The above results indicate that 5-ALA has a specific and strong inhibitory effect on SARS-CoV-2 infection and/or proliferation in various cell types.

## Claims

1. A therapeutic and/or preventive agent for coronavirus disease 2019 (COVID-19), the therapeutic and/or preventive agent comprising a compound or a salt of the compound, the compound represented by Formula (I) described below: 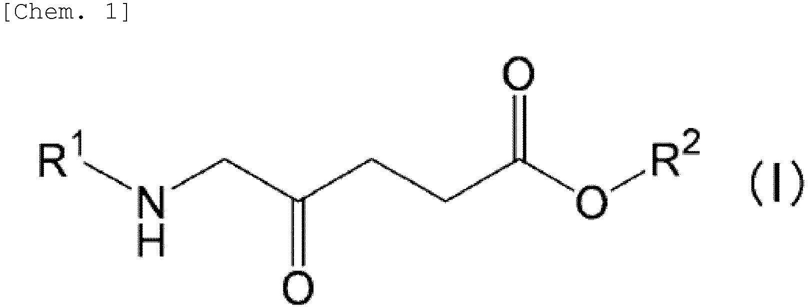 wherein R¹ represents a hydrogen atom or an acyl group, and R² represents a hydrogen atom, a linear or branched alkyl group, a cycloalkyl group, an aryl group, or an aralkyl group.

2. The therapeutic and/or preventive agent for coronavirus disease 2019 (COVID-19) according to claim 1, wherein R¹ and R² are respectively a hydrogen atom.

3. The therapeutic and/or preventive agent for coronavirus disease 2019 (COVID-19) according to claim 1 or 2, further comprising one or more metal-containing compounds.

4. The therapeutic and/or preventive agent for coronavirus disease 2019 (COVID-19) according to claim 3, wherein the one or more metal-containing compounds are a compound containing iron, magnesium, zinc, nickel, vanadium, copper, chromium, molybdenum, or cobalt.

5. The therapeutic and/or preventive agent for coronavirus disease 2019 (COVID-19) according to claim 3, wherein the one or more metal-containing compounds are a compound containing iron, magnesium, or zinc.

6. The therapeutic and/or preventive agent for coronavirus disease 2019 (COVID-19) according to claim 3, wherein the one or more metal-containing compounds are a compound containing iron.

7. The therapeutic and/or preventive agent for coronavirus disease 2019 (COVID-19) according to any one of claims 1 to 6, the therapeutic and/or preventive agent to be used in combination with a PPIX release inhibitor.

8. The therapeutic and/or preventive agent for coronavirus disease 2019 (COVID-19) according to claim 7, wherein the PPIX release inhibitor is a transporter inhibitor or an exocytosis inhibitor.

9. The therapeutic and/or preventive agent for coronavirus disease 2019 (COVID-19) according to claim 7 or 8, wherein the PPIX release inhibitor is an ABCG2 inhibitor or a dynamin inhibitor.

10. The therapeutic and/or preventive agent for coronavirus disease 2019 (COVID-19) according to any one of claims 7 to 9, wherein the PPIX release inhibitor is an ABCG2 inhibitor.
